# EUROPEAN PATENT APPLICATION

(11) **EP 4 552 608 A1**
(43) Date of publication of application: **14.05.2025**
(21) Application number: 23835718.0
(22) Date of filing: 22.06.2023
(51) Int. Cl.: A61C 7/00, A61C 7/08, A61C 9/00, A61B 5/00, G06T 17/00, G16H 50/50, G16H 30/00, B33Y 80/00, B33Y 70/00

(54) **METHOD FOR MANUFACTURING TRANSPARENT ORTHODONTIC DEVICE BY USING 3D PRINTER**

(30) Priority: 07.07.2022 KR 20220083852
(71) Applicant: ODS Co. Ltd., Incheon 21990 (KR)
(72) Inventor: PARK, Sungwon, Incheon 21990 (KR)
(74) Representative: Valet Patent Services Limited
(86) International application number: PCT/KR2023/008659
(87) International publication number: WO 2024/010251

(57) **Abstract**

This invention provides a more efficient, cost-effective, and streamlined method for manufacturing transparent orthodontic devices by utilizing 3D printing technology. It simplifies the production process, reduces collaboration time, and ensures that the aligners are customized for the patient's specific orthodontic needs.

## Description

### Technical Fields

The invention relates to manufacturing transparent orthodontic devices through the use of computers and 3D printers, specifically using a 3D scanner to capture the patient's oral structure, processing that data in a computer, and directly printing the transparent orthodontic devices with a 3D printer.

### Background Technology

The traditional method for producing dental braces is as follows:
First, the practitioner assesses the patient's oral structure and creates a dental model that matches the shape of the teeth. Typically, the patient's dental model is made by creating a mold of the oral structure, which is then used to produce a model, often using materials like plaster.

Next, the plaster model of the teeth is placed in a mold, and a sheet-like polyol material is heated and pressed in the vertical direction using a molding machine to create a transparent orthodontic appliance tailored to the patient. This conventional method of production involves collaboration between the dentist and the dental technician, with the process being manual and carried out by skilled dental technicians. As a result, it requires a significant amount of time and labor, which ultimately increases the cost of producing the orthodontic appliance.

On the other hand, recently, methods using 3D scanners to scan the patient's oral structure and store the information to create a dental model have become widely used. This method is an advanced approach compared to creating a dental model by directly taking an impression of the patient's teeth.

In other words, in dental clinics, the 3D scanning data of the patient's teeth is sent to the dental laboratory, which then uses the received information to create the dental model and, based on this, produce the transparent orthodontic appliance using the pressing method.

Furthermore, methods for producing dental models using 3D printers have also been disclosed. In this method, the dental data obtained from a 3D scanner is sent to a 3D printer, which then prints the patient's dental model.

Additionally, methods for directly producing transparent orthodontic appliances using 3D printers have been introduced. However, the above technologies are merely general and introductory methods.

### Detailed description of the Invention

### Technical Challenges

The method for manufacturing transparent orthodontic devices using a 3D printer according to the present invention aims to significantly reduce the time and labor required for the production of the orthodontic devices by directly printing the orthodontic appliance using a 3D printer, without the need to create a separate dental model. This approach dramatically lowers the unit cost of producing the orthodontic appliance.

Furthermore, the manufacturing method for the transparent orthodontic device according to the present invention eliminates the need for the inconvenient process of collaboration between dental clinics and dental laboratories. By enabling the direct production of orthodontic appliances in dental clinics, the invention simplifies the production process.

Additionally, the method for manufacturing the transparent orthodontic device using a 3D printer according to the present invention provides the optimal scale and thickness necessary to achieve the required corrective force for the orthodontic device, thus enhancing the comfort and effectiveness of the transparent orthodontic appliance.

### Technical Solution

To solve the above-mentioned problems, the method for manufacturing a transparent orthodontic device using a 3D printer according to the present invention includes the following steps:
(A) Scanning the patient's oral structure to generate and store data representing the shape of the oral structure,
(B) Displaying the patient's oral structure based on the data stored in step (A),
(C) Setting the detailed specifications of the orthodontic treatment based on the displayed patient's oral structure in step (B),

Step (C) includes the following stages: selecting the teeth to be treated while the patient's oral structure is displayed, inputting the correction values for the rotation and movement of the selected teeth, inputting the scale of the transparent orthodontic device based on the entered correction values, and inputting the thickness of the transparent orthodontic device to differentiate between areas that contact the treated teeth and areas that do not, thereby partially differentiating the thickness of the transparent orthodontic device.

The manufacturing method of the transparent orthodontic device using a 3D printer according to the present invention involves the transparent orthodontic device being printed through the 3D printer based on the set values as a solution to the problem.

Furthermore, the manufacturing method of the transparent orthodontic device using a 3D printer according to the present invention is characterized by the additional formation of a connection part on the outer surface and/or inner surface of the transparent orthodontic device that contacts the teeth to be treated, as a solution to the problem.

### Effects of the Invention

The method for manufacturing a transparent orthodontic device using a 3D printer according to the present invention significantly reduces the time and effort required to produce the orthodontic device by directly printing the device using a 3D printer, without the need to create a separate dental model. This enhances the production efficiency of the orthodontic device and improves the cost-effectiveness of the product.

Furthermore, the manufacturing method according to the present invention enables in-house manufacturing of the orthodontic device directly at dental clinics, simplifying the production process of the orthodontic device.

Additionally, by presenting the optimal scale and thickness required for securing the orthodontic force of the transparent orthodontic device, the method improves the user comfort and overall effectiveness of the transparent orthodontic device manufactured using a 3D printer.

### Brief Description og the Drawings

**Fig. 1** shows the time-dependent change in correction force as the scale of the aligner changes.
**Fig. 2** demonstrates the effect of the thickness of the aligner on the correction force over time.
**Fig. 3** shows a transparent orthodontic device with selective thickness adjustments for specific teeth.
**Fig. 4** illustrates an attachment on the aligner to improve correction force.
**Fig. 5** shows a connection part designed for additional correction force.

### Best Mode for Carrying Out the Invention

The manufacturing method of the transparent orthodontic device using a 3D printer according to the present invention includes the following steps:
(A) Scanning the patient's oral structure to generate and store data on the shape of the oral structure,
(B) Displaying the patient's oral structure based on the data stored in step (A), and
(C) Setting the detailed correction parameters for the patient's oral structure displayed in step (B).

Step (C) includes the following stages: selecting the teeth to be treated while the patient's oral structure is displayed, inputting the correction values for the rotation and movement of the selected teeth, inputting the scale of the transparent orthodontic device based on the entered correction values, and inputting the thickness of the transparent orthodontic device to differentiate between areas that contact the treated teeth and areas that do not, thereby partially differentiating the thickness of the transparent orthodontic device.

### Form for implementing the invention

Below is a detailed explanation of the steps involved in the method for manufacturing a transparent orthodontic device using a 3D printer according to the present invention.

The technical terms used in the present invention are merely for describing the following embodiments, and their use is not intended to limit the scope of the invention. Unless specifically defined otherwise, these terms should be understood to convey the meanings generally understood and used by those skilled in the art.

Step (A) is the stage where the patient's oral structure is scanned to generate and store data regarding the shape of the oral structure.

The practitioner scans the patient's oral structure using a 3D scanner, thereby generating data on the shape of each of the patient's teeth and storing this information on a computer.

Step (B) is the stage where the patient's oral structure is displayed based on the data stored in step (A). The practitioner reviews the displayed oral structure and formulates a treatment plan. Specifically, the practitioner identifies the teeth that require correction and establishes a correction plan for the selected teeth.

Step (C) is the stage where the detailed correction parameters are set for the patient's oral structure displayed in step (B). It consists of the following sub-steps Step (C1), Step (C2), Step (C3).

Step (C1) is the stage where, while the patient's oral structure is displayed, the teeth to be corrected are selected. The correction values for the rotation and movement of the selected teeth are then inputted.

Correction may involve rotating a tooth while keeping its position fixed (rotational movement), moving the tooth's position itself (horizontal/vertical movement), or a combination of both. The practitioner inputs the rotation values, movement directions, and movement values for each tooth based on the established treatment plan into the computer.

There are two ways to input this into the computer. The first method involves moving or rotating the teeth according to the correction plan while the oral structure is displayed. The second method involves inputting the numerical values for the movement and/or rotational movement. Regardless of the method used, both approaches would fall under one embodiment of the present invention.

Step (C2) is the stage where the scale of the transparent orthodontic device is inputted based on the correction values entered in the previous step. The scale refers to the ratio of the transparent orthodontic device's volume relative to the tooth's volume.

Fundamentally, the transparent orthodontic device is made to fit the corrected state of the treated teeth. In this case, the scale of the transparent orthodontic device directly affects the orthodontic force applied to the teeth.

If the scale is too small (meaning the transparent orthodontic device closely matches the hypothetical corrected size of the teeth, leaving almost no gap between the teeth and the aligner), it may cause difficulty in wearing the aligner or lead to severe discomfort for the patient. On the other hand, if the scale is too large (meaning the gap between the teeth and the aligner increases), the aligner may be easier to wear but would not apply sufficient corrective force, leading to reduced efficacy.

The correction method using the transparent orthodontic device works by gradually adjusting the teeth. Once the desired position or rotation is reached, the patient replaces the aligner with the next stage, and further correction occurs with the new aligner. Thus, the transparent orthodontic device needs to be replaced at regular intervals, following a staged process of treatment.

At the beginning of treatment, when the first aligner is worn, the correction force is at its maximum. However, as time passes and the teeth gradually shift, the orthodontic force exerted by the aligner gradually decreases. This necessitates changing the aligners at intervals to continue the correction process effectively.

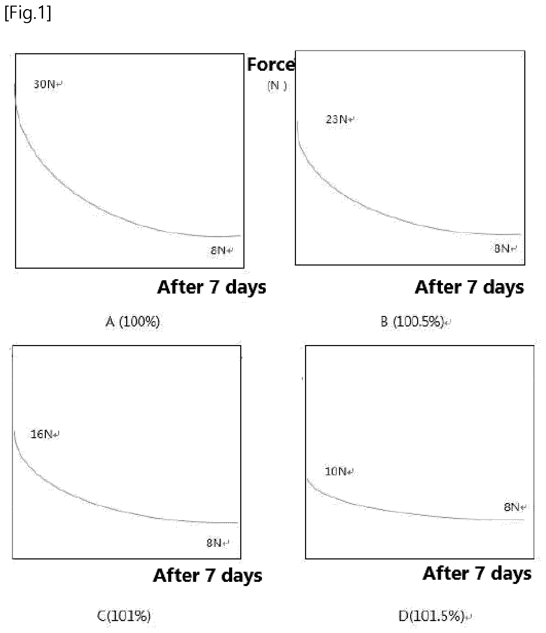

The effect of the scale on correction force is visually represented in **Figure 1****,** which shows how the correction force changes in relation to the scale of the aligner over time.

If the scale is set to 100% (where the shape of the teeth after correction perfectly matches the size of the transparent orthodontic device), the maximum correction force is achieved. However, this can result in poor wearability, and in some cases, it may even be impossible to wear the device.

On the other hand, increasing the scale reduces the correction force. If the scale value exceeds 101.5%, the correction force significantly decreases. Therefore, the scale value of the transparent orthodontic device should not exceed 101.5%. Additionally, to ensure some level of comfort and usability, the scale value should be between 100.1% and 101.5%. This range is recommended to balance both correction force and wearability.

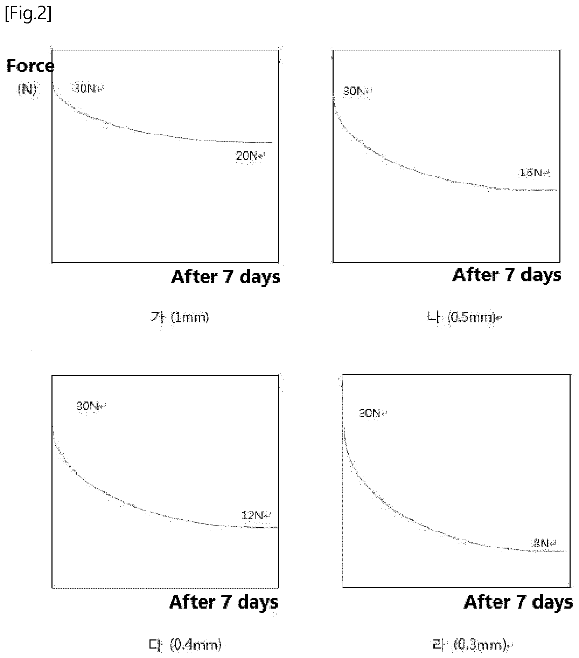

Step (C3) is the stage where the thickness of the transparent orthodontic device is differentiated by adjusting the areas that contact the treated teeth and the areas that do not, thereby inputting a partially varied thickness for the transparent orthodontic device. Figure 2 shows the time variation of the correction force based on the thickness of the transparent orthodontic device.

In order to apply a force to the teeth and induce changes in their position, the thickness of the transparent orthodontic device must be above a certain threshold value. The transparent orthodontic device will only be effective in achieving the desired correction if its thickness exceeds this minimum required value.

However, if the thickness of the transparent orthodontic device is increased solely to ensure sufficient corrective force, it may result in a significant decrease in comfort, causing discomfort for the patient. Furthermore, beyond a certain thickness, additional corrective force will no longer be effective, and no further improvements will be gained by increasing the thickness.

As shown in Figure 2, the minimum thickness of the transparent orthodontic device that maintains a certain level of correction force during the typical replacement cycle of 7 to 14 days (at the part contacting the target teeth) is 0.3 mm. If the thickness of the device exceeds 1 mm, patients experience considerable discomfort, and additional correction force is not effectively applied. Therefore, it is preferable for the thickness of the transparent orthodontic device at the contact points with the target teeth to be at least 0.3 mm but not exceed 1 mm. Ideally, the thickness should range from 0.3 mm to 0.7 mm.

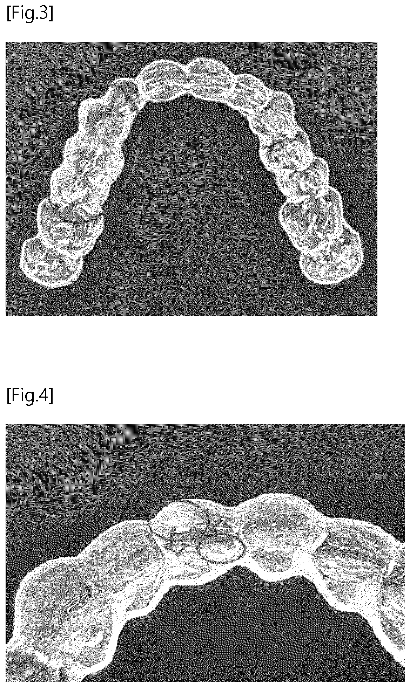

Figure 3 illustrates an embodiment of the present invention, where the thickness of the transparent orthodontic device is increased only at the parts of the device that correspond to the teeth requiring correction.

The transparent orthodontic device according to the present invention allows for adjusting the thickness specifically at the contact areas with the teeth requiring correction. In other words, the thickness of the transparent orthodontic device is increased at the parts that come into contact with the target teeth, thereby increasing the rigidity of the device and enabling a greater corrective force.

Step (D) is the process of completing the transparent orthodontic device according to the present invention by printing it through a 3D printer based on the previously set values.

The transparent orthodontic device according to the present invention is characterized by using a low-viscosity resin as the material for 3D printing, which results in a precise device that also possesses the required strength and elasticity.

If the viscosity of the resin is too high, its workability during molding is compromised, leading to difficulties in 3D printing and potentially causing equipment malfunctions. Additionally, high viscosity can cause material separation, impairing the uniformity of the product and resulting in defects. To address these issues, the present invention uses a low-viscosity resin in the 3D printing process.

For these reasons, it is preferable to limit the viscosity of the resin used in the 3D printer to below 750 cPs (centi-poise). However, if the viscosity is too low, the rigidity (stiffness) of the finished transparent orthodontic device may fall below the required levels, weakening its corrective force.

Therefore, it is preferable for the viscosity of the resin used in the 3D printer to be at least 650 cPs or higher.

Thus, it is desirable for the viscosity of the resin to range from 650 cPs to 750 cPs. More preferably, the viscosity of the resin should be between 675 cPs and 725 cPs. This ensures that the transparent orthodontic device has the necessary physical properties(strength and elasticity) while also being efficiently printable using a 3D printer.

Step (E) involves inputting the surface area, thickness, shape, etc., of an attachment that will be formed on the inner surface of the transparent orthodontic device in contact with the target teeth, in order to exert a stronger corrective force. Figure 4 illustrates an embodiment of the transparent orthodontic device according to the present invention, with the attachment formed.

By creating an attachment, such as a protrusion, on the inner surface of the device in contact with the target teeth, a stronger pressure can be applied to the teeth, thereby doubling the corrective force of the transparent orthodontic device and enhancing its overall effectiveness. The corrective force varies depending on the thickness, surface area, shape, etc., of the attachment, so these factors are also input based on the set corrective values.

Figure 4 shows an attachment specifically designed for rotating the target teeth, but this is merely an example of the present invention. Depending on the orthodontic plan for the teeth, the number, size, and shape of the attachments can be adjusted as needed.

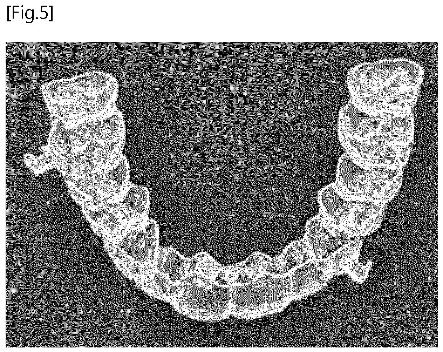

Figure 5 illustrates an embodiment of the transparent orthodontic device according to the present invention, featuring the connection portion described above. Step (F) involves inputting a connection portion on the outer surface of the transparent orthodontic device, in contact with the target teeth, to secure additional corrective force. The connection portion is formed either on the outer or inner surface of the transparent orthodontic device. In cases requiring a stronger corrective force, the orthodontic wire can be attached to the connection portion to enhance the corrective force.

The shape of the connection portion can vary, such as a hook shape or button shape, as long as it serves the purpose of attaching an orthodontic wire. These variations are considered simple modifications of the present invention and fall within its technical scope.

The description of the configuration and effects as described above is merely an embodiment of the present invention and does not limit the scope of the claims of the invention. Various modifications and changes can be made within the scope of the technical spirit of the present invention, which will be apparent to those skilled in the art. Such simple design modifications will be considered to fall within the technical scope of the present invention.

## Claims

1. In a method for manufacturing a transparent orthodontic appliance using a computer, the method comprising the steps of:
(A) Scanning the patient's oral structure to generate and store data representing the shape of the oral structure;
(B) Displaying the patient's oral structure based on the data stored in step (A); and
(C) Setting the detailed aspects of the orthodontic treatment with respect to the displayed oral structure in step (B),
wherein step (C) includes:
Selecting the teeth to be treated while the patient's oral structure is displayed and entering correction values for the rotation and movement amounts of the selected teeth: (C1)Entering a scale value representing the gap between the orthodontic appliance and the teeth based on the correction values entered, wherein the scale value entered is between 100.1% and 101.5%: (C2)Entering thickness values to differentially vary the thickness of the orthodontic appliance by differentiating the thickness at the parts that contact the orthodontic appliance with the teeth and the parts that do not: (C3)
(D) Outputting the transparent orthodontic appliance through a 3D printer based on the values set in the above steps.

2. The method of claim 1, wherein step (E) further includes inputting at least one of the surface area, thickness, or shape of an attachment formed on the inner surface of the transparent orthodontic appliance, which provides stronger orthodontic force at the contact area with the teeth.

3. The method of claim 1, wherein step (C) further includes:
for securing additional orthodontic force, inputting a connection part on the outer or inner surface of the transparent orthodontic appliance that contacts the teeth to secure the orthodontic force: (F).

4. The method of claim 2, wherein the thickness value entered for the portion that contacts the teeth in step (C3) is between 0.3mm and 0.7mm.

5. The method of claim 2, wherein the connection part is in the form of a hook or a protruding button.

6. The method of claim 3, wherein the connection part is in the form of a hook or a protruding button.
